# EUROPÄISCHE PATENTSCHRIFT

(11) **EP 0 863 125 B1**
(45) Veröffentlichungstag und Bekanntmachung des Hinweises auf die Patenterteilung: **28.11.2001**
(21) Anmeldenummer: 98103245.1
(22) Anmeldetag: 25.02.1998
(51) Int. Cl.: C07C 67/31, C07C 69/675, C07D 339/04

(54) **Verfahren zur Herstellung von enantiomerenreinen 3-Hydroxyoctandisäurediestern durch asymmetrische katalytische Hydrierung**
Process for the preparation of enantiomerically pure diesters of 3-hydroxyoctandioic acid by asymmetric catalytic hydrogenation
Procédé de préparation de diesters enantiomériquement pures de l'acide 3-hydroxyoctandioique par hydrogénation catalytique asymmétrique

(30) Priorität: 06.03.1997 DE 19709069
(43) Veröffentlichungstag der Anmeldung: 09.09.1998
(73) Patentinhaber: ASTA Medica Aktiengesellschaft, 01277 Dresden (DE)
(72) Erfinder: Gewald, Rainer, Dr., 01217 Dresden (DE); Laban, Gunter, Dr., 01465 Langebrück (DE)
(74) Vertreter: Wibbelmann, Jobst, Dr., Dipl.-Chem.

(56) Entgegenhaltungen:
- EP-A- 0 295 109
- EP-A- 0 487 986
- WO-A-95/18784
- AKIRA TAI ET AL.: "Highly Efficient Enantio-differentiating Hydrogenation over an Ultrasonicated Raney Nickel Catalyst Modified with Tartaric Acid" CHEMICAL COMMUNICATIONS., Nr. 12, 15.Juni 1991, CIETY OF CHEMISTRY GB, Seiten 793-796, XP002066441
- AKIRA TAI ET AL.: "An Improved Asymmetrically-Modified Nickel Catalyst Prepared from Ultrasonicated Raney Nickel" BULLETIN OF THE CHEMICAL SOCIETY OF JAPAN., Bd. 67, Nr. 9, September 1994, TOKYO JP, Seiten 2473-2477, XP002066442

## Beschreibung

Die vorliegende Erfindung betrifft ein neues Verfahren zur Herstellung von enantiomerenreinen 3-Hydroxyoctandisäurediestern der allgemeinen Formel I, wobei R¹ und R² gleich oder verschieden sind und eine C₁-C₂₀-Alkylgruppe, C₃-C₁₂-Cycloalkylgruppe, C₇-C₁₂-Aralkylgruppe oder eine ein- oder zweikernige Arylgruppe bedeuten.

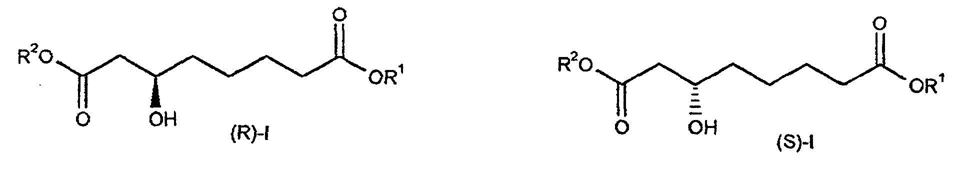

Die Verbindungen (R)-I sind neu, während die Verbindungen (S)-I bekannt sind. Beide dienen vornehmlich als Zwischenprodukte für die Synthese von enantiomerenreiner α-Liponsäure der Formel II und ihrer Derivate. α-Liponsäure ist 1,2-Dithiolan-3-pentansäure (Thioctsäure).

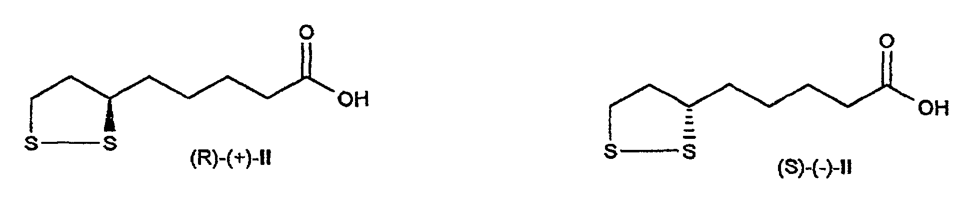

Das (R)-Enantiomer der α-Liponsäure (R)-(+)-II ist ein Naturstoff, der in geringen Konzentrationen in praktisch allen tierischen und pflanzlichen Zellen vorkommt. Als Coenzym bei der oxidativen Decarboxylierung von α-Ketocarbonsäuren (z.B. Brenztraubensäure) ist α-Liponsäure von essentieller Bedeutung. α-Liponsäure ist pharmakologisch wirksam und weist antiphlogistische und antinociceptive (analgetische) sowie zytoprotektive Eigenschaften auf. Eine wichtige medizinische Indikation ist die Behandlung der diabetischen Polyneuropathie. Nach neueren Ergebnissen (A. Baur et al., Klin. Wochenschr. 1991, 69, 722; J.P. Merin et al., FEBS Lett. 1996, 394, 9) kann α-Liponsäure möglicherweise Bedeutung bei der Bekämpfung durch HIV-1- und HTLV IIIB-Viren bedingter Krankheiten erlangen.

Bei den reinen optischen Isomeren der α-Liponsäure (R- und S-Form, d.h. (R)-α-Liponsäure und (S)-α-Liponsäure) ist im Gegensatz zu dem Razemat das (R)-Enantiomer vorwiegend antiphlogistisch und das (S)-Enantiomer vorwiegend antinociceptiv wirksam (EP 0427247, 08.11.90). Unterschiedliche pharmakokinetische Eigenschaften der beiden Enantiomere sind ebenfalls festgestellt worden (R. Hermann et al., Eur. J.Pharmaceut. Sci. 1996, 4, 167). Daher ist die Synthese der reinen Enantiomere von großer Wichtigkeit.

Bekannte Herstellungsverfahren der enantiomerenreinen α-Liponsäuren umfassen die Razematspaltung der α-Liponsäure oder ihrer Vorstufen, asymmetrische Synthesen unter Einsatz chiraler Auxilarien, "chiral pool"-Synthesen unter Verwendung von in der Natur vorkommenden optisch aktiven Ausgangsverbindungen sowie mikrobielle Synthesen (Übersichtsartikel: J.S. Yadav et al., J. Sci. Ind. Res. 1990, 49, 400; sowie: E. Walton et al., J. Am. Chem. Soc. 1955, 77, 5144; D.S. Acker und W.J. Wayne, J. Am. Chem. Soc. 1957, 79, 6483; L.G. Chebotareva und A.M. Yurkevich, Khim.-Farm. Zh. 1980, 14, 92; A.S. Gopalan et al., Tetrahedron Lett. 1989, 5705; A.G. Tolstikov et al., Bioorg. Khim. 1990, 16, 1670; L. Dasaradhi et al., J. Chem. Soc., Chem. Commun. 1990, 729; A.S. Gopalan et al., J. Chem. Perkin Trans. 1 1990, 1897; EP 0487986 A2, 14.11.91; B. Adger et al., J. Chem. Soc., Chem. Commun. 1995, 1563; Y.R. Santosh Laxmi und D.S. lyengar, Synthesis, 1996, 594).

Davon stellt die Razematspaltung über die Bildung von diastereomeren Salzen der α-Liponsäure mit optisch aktivem α-Methylbenzylamin (DE-OS 4137773.7, 16.11.91 und DE-OS 4427079.8, 30.07.94) die bisher wirtschaftlichste Variante dar. Da die Razemattrennung erst auf der letzten Stufe der Synthesesequenz erfolgt, sind jedoch keine hohen Ausbeuten zu erzielen.

Das einzige bekannte chemokatalytische asymmetrische Verfahren zur Herstellung von enantiomerenreiner α-Liponsäure (DE-OS 3629116.1, 27.08.86) beruht auf der Sharpless-Epoxydation von Allylalkoholen, ist aber wegen der hohen Kosten der Ausgangsverbindungen unwirtschaftlich.

Unter den beschriebenen biokatalytischen Synthesewegen ist die asymmetrische Reduktion von 3-Oxo-octandisäurediestern III mit Bäckerhefe hervorzuheben (EP 0487986 A2, 14.11.91). Die Nachteile dieses Verfahrens bestehen jedoch darin, daß die Raum-Zeit-Ausbeute äußerst gering ist, ein hoher Enantiomerenüberschuß nur bei Einsatz des Isobutylesters (R¹=iBu) erreicht werden kann und stets überwiegend das (S)-Enantiomer (S)-I gebildet wird.

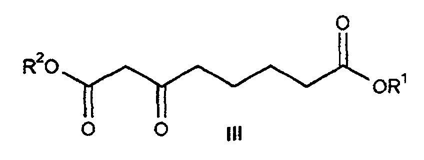

Aufgabe der Erfindung ist es deshalb, wahlweise beide Enantiomere der α-Liponsäure in hoher chemischer und optischer Raum-Zeit-Ausbeute bei Verwendung kostengünstiger Ausgangsstoffe zugänglich zu machen. Erfindungsgemäß gelingt dies durch asymmetrische chemokatalytische Hydrierung von 3-Oxo-octandisäurediestem der Formel III, bei der R¹ und R² jeweils unabhängig voneinander eine C₁-C₂₀-Alkylgruppe, C₃-C₁₂-Cycloalkylgruppe, C₇-C₁₂-Aralkylgruppe oder eine ein- oder zweikernige Arylgruppe bezeichnen, in Gegenwart von Komplexen aus Ruthenium und optisch aktiven Phosphinen bzw. aus Raney-Nickel und optisch aktiver Weinsäure als Katalysatoren.

Dabei werden unabhängig von der Art der Estergruppen (R¹, R²) gleichbleibende hohe optische und chemische Ausbeuten an 3-Hydroxyoctandisäurediestem I erzielt. Im Gegensatz zur biokatalytischen Variante kann bei sehr hohen Substratkonzentrationen gearbeitet werden.

Die Verbindungen III sind bekannt und vor allem durch Acylierung von Meldrum-Säure mit Adipinsäuremonoalkylesterchlorid und anschließender Alkoholyse erhältlich (H. Thoma und G. Spiteller, Liebigs Ann. Chem. 1983, 1237; EP 0487986 A2, 14.11.91). Unter bestimmten Reaktionsbedingungen lassen sich erfindungsgemäß vorzugsweise auch die vor der Alkoholyse intermediär gebildeten und isolierbaren 6-(2,2-Dimethyl-4,6-dioxo-1,3-dioxan-5-yliden)-6-hydroxy-hexansäurealkylester der Formel IV (R¹= C₁-C₂₀-Alkyl, C₃-C₁₂-Cycloalkyl, C₇-C₁₂-Aralkyl und/oder ein- oder zweikemiges Aryl) zur asymmetrischen Hydrierung einsetzen. Sie lassen sich wie beschrieben (H.W. Schmidt und M. Klade, Org. Prep. Proced. Int. 1988, 20, 184) oder in analoger Weise darstellen.

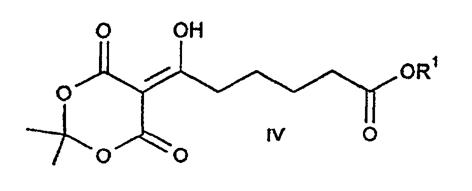

Von besonderem Interesse als Katalysatoren für die asymmetrische Hydrierung sind Ruthenium-Diphosphin-Komplexe. Als typisch aber nicht als Einschränkung seien die Rutheniumkomplexe der folgenden Formeln V bis XI genannt:

[RuHal₂D]_{1,2}(L)ₓ V

[RuHalAD]⁺Y⁻ VI

RuDₙOOCR³OOCR⁴ VII

[RuHₓDₙ]^{m+}Yₘ⁻ VIII

[RuHal(PR⁵ ₂R⁶)D]²⁺Hal₂⁻ XI

[RuHHalD₂] X

[DRu(acac)₂] XI

worin:
- acac: für Acetylacetonat steht,
- D: für ein Diphosphin der allgemeinen Formel XII steht,
- Hal: für Halogen, insbesondere lod, Chlor oder Brom steht,
- R³ und R⁴: gleich oder verschieden sind und für Alkyl mit bis zu 9 C-Atomen, vorzugsweise bis zu 4 C-Atomen, welches gegebenenfalls substituiert ist durch Halogen, insbesondere Fluor, Chlor oder Brom oder für Phenyl stehen, welches gegebenenfalls durch Alkyl mit 1 bis 4 C-Atomen substituiert ist oder für eine α- Aminoalkylsäure mit vorzugsweise bis zu 4 C-Atomen stehen, oder gemeinsam eine Alkylidengruppe mit bis zu 4 C-Atomen bilden,
- R⁵ und R⁶: jeweils gleich oder verschieden sind und für gegebenenfalls substituiertes Phenyl stehen, vorzugsweise substituiert durch Alkyl mit 1 bis 4 C-Atomen oder Halogen,
- Y: für Cl, Br, J, ClO₄, BF₄ oder PF₆ stehen,
- A: für einen unsubstituierten oder substituierten Benzolring wie p-Cymol steht.
- L: für einen neutralen Liganden wie Aceton, ein tertiäres Amin oder Dimethylformamid steht,
- n und m: jeweils für 1 oder 2 stehen,
- x: für 0 oder 1 steht,
wobei in Formel VIII n für 1 und m für 2 steht, wenn x=0 bedeutet, und n für 2 und m für 1 steht, wenn x=1 bedeutet.

Die Komplexe der Formeln V bis XI können nach an sich bekannten Methoden hergestellt werden (V und X: EP 174057 und J.P. Genet et al., Tetrahedron Asymmetry 1994, 5, 675; VI: EP 366390; VII: EP 245959 und EP 272787; VIII: EP 256634; IX: EP 470756; XI: P. Stahly et al., Organometallics 1993,1467).

Als optisch aktive Diphosphin-Liganden kommen Verbindungen der allgemeinen Formel XII zur Anwendung: 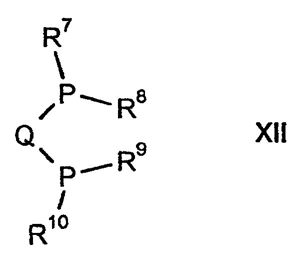 worin:
- Q: für eine die beiden P-Atome verbrückende Gruppe mit 2 bis 24 Kohlenstoffatomen und gegebenenfalls 1 bis 4 Heteroatomen, vorzugsweise O, S, N und Si, steht, wobei die Verbrückung von mindestens 2 der Kohlenstoffatome und gegebenenfalls 1 bis 4 der Heteroatome gebildet wird,
- R⁷- R¹⁰: jeweils gleich oder verschieden sind und für Alkylgruppen mit 1 bis 18 C-Atomen, Cycloalkylgruppen mit 5 bis 7 C-Atomen oder Arylgruppen mit 6 bis 12 C-Atomen stehen.

Als besonders bevorzugte, in enantiomerenreiner Form zum Einsatz kommende chirale Diphosphine können folgende Liganden als Beispiele aufgeführt werden:

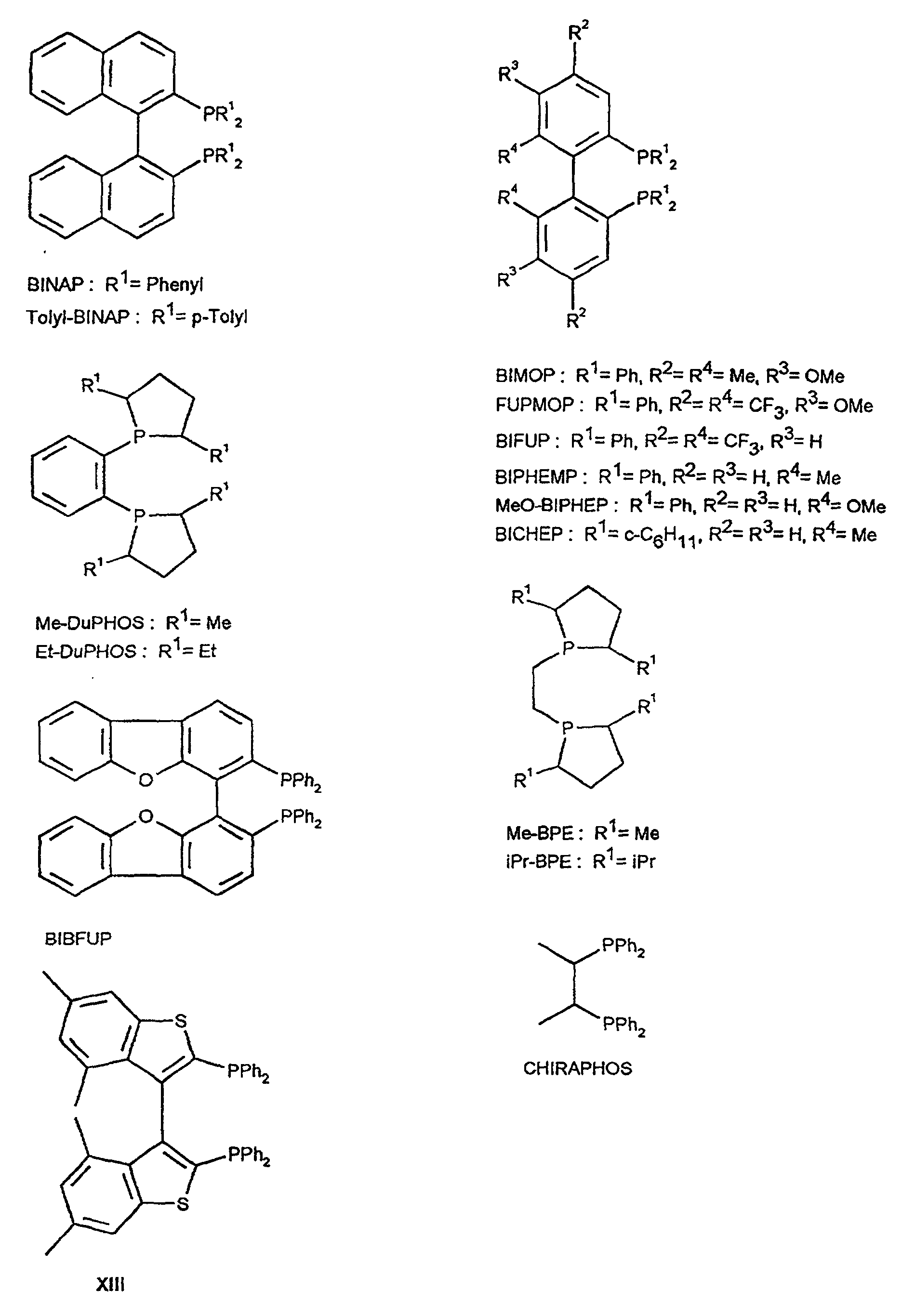

Die oben der Einfachheit halber als razemische Strukturen aufgeführten Liganden sind in ihren enantiomerenreinen Formen bekannte Verbindungen (BINAP: R. Noyori et al., J. Am. Chem. Soc. 1980, 102, 7932; BIMOP, FUPMOP, BIFUP: M. Murata et al., Synlett 1991,827; BIBHEMP: R. Schmid et al., Helv. Chim. Acta 1988, 71, 697; MeO-BIPHEP: R. Schmid et al., Helv. Chim. Acta 1991, 74, 370; BICHEP: A. Miyashita et al., Chem. Lett. 1989, 1849; DuPHOS: M. Burk et al., Organometallics 1990, 9, 2653; BPE: M. Burk et al., J. Am. Chem. Soc. 1995, 117, 4423; BIBFUP: EP 643065; CHIRAPHOS: B. Bosnich et al., J. Am. Chem. Soc. 1977, 99, 6262; XIII: WO 96/01831).

Die asymmetrische Hydrierung der Verbindungen der Formel III in Gegenwart der oben beschriebenen optisch aktiven Ruthenium-Diphosphin-Komplexen der Formeln V bis XI kann in geeigneten, unter den Reaktionsbedingungen inerten organischen Lösungsmitteln durchgeführt werden. Als solche können insbesondere genannt werden, Alkohole wie Methanol oder Ethanol, chlorierte Kohlenwasserstoffe wie Methylenchlorid oder Dichlorethan, cyclische Ether wie Tetrahydrofuran oder Dioxan, Ester wie z.B. Essigester, aromatische Kohlenwasserstoffe wie Benzol oder Toluol, oder auch Gemische hiervon und dergleichen. Zur Unterdrückung einer möglichen Ketalbildung beim Arbeiten in Alkoholen als Lösungsmittel können bis zu 10 Vol.-% Wasser zugesetzt werden. Die Substratkonzentrationen liegen vorzugsweise bei 5 bis 50 Vol.-%, insbesondere bei 20 bis 40 Vol.-%.

Die Umsetzungen können vorzugsweise bei Temperaturen von etwa 10°C bis 120°C, insbesondere von etwa 20°C bis 70°C und unter einem Wasserstoffdruck von etwa 1 bis 100 bar, insbesondere von 4 bis 50 bar, durchgeführt werden. Die Reaktionszeiten betragen im allgemeinen 2 bis 48 Stunden, meistens 6 bis 24 Stunden. Das molare Verhältnis zwischen Ruthenium in den Komplexen V bis XI und den zu hydrierenden Verbindungen III liegt zweckmäßig zwischen etwa 0,001 und etwa 5 Mol.-%, vorzugsweise zwischen etwa 0,005 und etwa 0,2 Mol.-%.

Unter den oben genannten Bedingungen lassen sich erfindungsgemäß auch Verbindungen der Formel IV in Gegenwart der optisch aktiven Ruthenium-Diphosphin-Komplexe der Formeln V bis XI asymmetrisch hydrieren, wobei die Umsetzung vorzugsweise in Alkoholen oder Gemischen aus oben genannten organischen Lösungsmitteln und mindestens 1, vorzugsweise 4 bis 10 mol Alkohol, bezogen auf die zu hydrierende Verbindung IV und bei Temperaturen von etwa 40°C bis 120°C, insbesondere von etwa 50°C bis 100°C, durchgeführt wird. In den Reaktionsprodukten ist dann der Rest R² durch den entsprechenden eingesetzten Alkohol festgelegt.

In der Reaktion kann das gewünschte Enantiomer der Formel I durch Auswahl des optisch aktiven Diphosphinliganden der Formel XII mit der entsprechenden Konfiguration erhalten werden. So führt beispielsweise die Verwendung von (R)-(+)-BINAP zu Produkten der Formel (R)-I, der Einsatz von (S)-(-)-BINAP zu Produkten der Formel (S)-I.

Die Verbindungen I dienen zur Herstellung der enantiomerenreinen α-Liponsäuren der Formel II, indem sie auf bekanntem Wege (EP 0487986 A2, 14.11.91) zu den Verbindungen XIV, wobei R¹ eine C₁-C₂₀-Alkylgruppe, C₃-C₁₂-Cycloalkylgruppe, C₇-C₁₂-Aralkylgruppe oder eine ein- oder zweikemige Arylgruppe bedeutet, reduziert werden 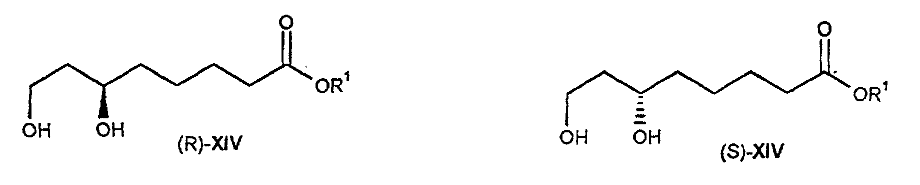 und diese
a) in organischer Lösung mit einem Sulfonsäurechlorid und einer tertiären Stickstoffbase in den Bissulfonsäureester von XIV überführt werden,
b) diese Verbindung in einem polaren Lösungsmittel mit Schwefel und einem Alkalimetallsulfid zum α-Liponsäureester umgesetzt wird und
c) dieser Ester gewünschtenfalls in das jeweilige reine Enantiomer der α-Liponsäure überführt wird. Dabei wird ausgehend von den Verbindungen (R)-I die (S)-(-)-α-Liponsäure und ausgehend von den Verbindungen (S)-I die (R)-(+)-α-Liponsäure erhalten.

Die nach dem erfindungsgemäßen Verfahren hergestellten Verbindungen (R)-I und (S)-I sowie (R)-(+)-II und die (S)-(-)-II weisen in der Regel einen hohen Enantiomerenüberschuß auf, entsprechend einer optischen Ausbeute von 70 bis 99%.

Die Enantiomerenverhältnisse werden direkt durch chirale HPLC an optisch aktiven Säulen gemessen.

Die vorliegende Erfindung ermöglicht es, die enantiomerenreinen 3-Hydroxyoctandisäurediester der allgemeinen Formel I (R¹,R² = C₁-C₂₀-Alkyl, C₃-C₁₂-Cycloalkyl, C₇-C₁₂-Aralkyl und/oder ein- oder zweikemiges Aryl) als Zwischenprodukte zur Herstellung der enantiomerenreinen α-Liponsäuren der Formel II auf wirtschaftliche Weise in hohen chemischen und optischen Ausbeuten zugänglich zu machen.

Die nachfolgenden Beispiele erläutern die Erfindung, ohne diese zu beschränken.

### Beispiel 1

In ein 20 ml-Schlenkgefäß wurden unter Argon 43,5 mg (0,087 mmol) [RuCl₂(C₆H₆)]₂, 113,7 mg (0,183 mmol) (R)-BINAP und 3 ml Dimethylformamid gegeben. Die rötlichbraune Suspension wurde 10 min auf 100°C erhitzt. Die nunmehr klare Lösung wurde abgekühlt und im Vakuum (1 bis 0,1 mmHg) bei 50°C unter starkem Rühren über einen Zeitraum von 1 h eingeengt. Der verbliebene orangebraune Feststoff wurde in 1 ml Tetrahydrofuran aufgenommen und kam so als Ru-(R)-BINAP-Katalysator in den asymmetrischen Hydrierungen zum Einsatz.

### Beispiel 2

In ein 20 ml-Schlenkgefäß wurden unter Argon 43,5 mg (0,087 mmol [RuCl₂(C₆H₆)]₂, 113,7 mg (0,183 mmol) (S)-BINAP und 3 ml Dimethylformamid gegeben. Die rötlichbraune Suspension wurde 10 min auf 100°C erhitzt. Die nunmehr klare Lösung wurde abgekühlt und im Vakuum (1 bis 0,1 mmHg) bei 50°C unter starkem Rühren über einen Zeitraum von 1 h eingeengt. Der verbliebene orangebraune Feststoff wurde in 1 ml Tetrahydrofuran aufgenommen und kam so als Ru-(S)-BINAP-Katalysator in den asymmetrischen Hydrierungen zum Einsatz.

### Beispiel 3

In ein 20 ml-Schlenkgefäß wurden unter Argon 43,5 mg (0,087 mmol) [RuCl₂(C₆H₆)]₂, 124,2 mg (0,183 mmol) (R)-Tolyl-BINAP und 3 ml Dimethylformamid gegeben. Die rötlichbraune Suspension wurde 10 min auf 100°C erhitzt Die nunmehr klare Lösung wurde abgekühlt und im Vakuum (1 bis 0,1 mmHg) bei 50°C unter starkem Rühren über einen Zeitraum von 1 h eingeengt. Der verbliebene orangebraune Feststoff wurde in 1 ml Tetrahydrofuran aufgenommen und kam so als Ru-(R)-Tolyl-BINAP-Katalysator in den asymmetrischen Hydrierungen zum Einsatz.

### Beispiel 4

Ein 100 ml-Autoklav wurde unter Argon mit 21,6 g (0,1 mol) 3-Oxooctandisäuredimethylester, mit der unter Beispiel 1 hergestellten Ru-(R)-BINAP-Katalysatorlösung und mit 40 ml sauerstofffreiem Methanol beladen. Die Hydrierung wurde bei 60°C, einem konstanten Druck von 40 bar reinem H₂ und unter intensivem Rühren 20 Stunden durchgeführt. Nach Beendigung der Reaktion wurde das Lösungsmittel am Rotationsverdampfer abdestilliert. Man erhielt 21,2 g (97%) (R)-3-Hydroxyoctandisäuredimethylester (Gehalt: 96%) mit einem Enantiomerenüberschuß von 98% (chirale HPLC).

### Beispiel 5

Ein 100 ml-Autoklav wurde unter Argon mit 21,6 g (0,1 mol) 3-Oxooctandisäuredimethylester, mit der unter Beispiel 2 hergestellten Ru-(S)-BINAP-Katalysatorlösung und mit 40 ml sauerstofffreiem Methanol beladen. Die Hydrierung wurde bei 65°C, einem konstanten Druck von 35 bar reinem H₂ und unter intensivem Rühren 20 Stunden durchgeführt. Nach Beendigung der Reaktion wurde das Lösungsmittel am Rotationsverdampfer abdestilliert. Man erhielt 21,3 g (98%) (S)-3-Hydroxyoctandisäuredimethylester (Gehalt: 97%) mit einem Enantiomerenüberschuß von 98% (chirale HPLC).

### Beispiel 6

Ein 100 ml-Autoklav wurde unter Argon mit 21,6 g (0,1 mol) 3-Oxooctandisäuredimethylester, mit der unter Beispiel 3 hergestellten Ru-(R)-Tolyl-BINAP-Katalysatorlösung und mit 40 ml sauerstofffreiem Methanol beladen. Die Hydrierung wurde bei 65°C, einem konstanten Druck von 40 bar reinem H₂ und unter intensivem Rühren 20 Stunden durchgeführt. Nach Beendigung der Reaktion wurde das Lösungsmittel am Rotationsverdampfer abdestilliert. Man erhielt 21,2 g (97%) (R)-3-Hydroxyoctandisäuredimethylester (Gehalt: 97%) mit einem Enantiomerenüberschuß von 97% (chirale HPLC).

### Beispiel 7

Ein 100 ml-Autoklav wurde unter Argon mit 14,3 g (0,05 mol) 6-(2,2-Dimethyl-4,6-dioxo-1,3-dioxan-5-yliden)-6-hydroxyhexansäuremethylester, mit der unter Beispiel 1 hergestellten Ru-(R)-BINAP-Katalysatorlösung und mit 40 ml sauerstofffreiem Methanol beladen. Die Hydrierung wurde bei 70°C, einem konstanten Druck von 50 bar reinem H₂ und unter intensivem Rühren 20 Stunden durchgeführt. Nach Beendigung der Reaktion wurde das Lösungsmittel am Rotationsverdampfer abdestilliert. Man erhielt 10,4 g (95%) (R)-3-Hydroxyoctändisäuredimethylester (Gehalt: 96%) mit einem Enantiomerenüberschuß von 97% (chirale HPLC).

### Beispiel 8

Ein 100 ml-Autoklav wurde unter Argon mit 14,3 g (0,05 mol) 6-(2,2-Dimethyl-4,6-dioxo-1,3-dioxan-5-yliden)-6-hydroxyhexansäuremethylester, mit der unter Beispiel 1 hergestellten Ru-(R)-BINAP-Katalysatorlösung und mit 40 ml sauerstofffreiem Ethanol beladen. Die Hydrierung wurde bei 70°C, einem konstanten Druck von 50 bar reinem H₂ und unter intensivem Rühren 20 Stunden durchgeführt. Nach Beendigung der Reaktion wurde das Lösungsmittel am Rotationsverdampfer abdestilliert. Man erhielt 11,1 g (96%) (R)-1-Ethyl-3-hydroxy-8-methyl-octandioat (Gehalt: 95%) mit einem Enantiomerenüberschuß von 98% (chirale HPLC).

### Beispiel 9

Ein 100 ml-Autoklav wurde unter Argon mit 24,4 g (0,1 mol) 3-Oxooctandisäurediethylester, mit der unter Beispiel 2 hergestellten Ru-(S)-BINAP-Katalysatorlösung und mit 40 ml sauerstofffreiem Methanol beladen. Die Hydrierung wurde bei 60°C, einem konstanten Druck von 30 bar reinem H₂ und unter intensivem Rühren 24 Stunden durchgeführt. Nach Beendigung der Reaktion wurde das Lösungsmittel am Rotationsverdampfer abdestilliert. Man erhielt 23,9 g (97%) (S)-3-Hydroxyoctandisäurediethylester (Gehalt: 98%) mit einem Enantiomerenüberschuß von 98% (chirale HPLC).

### Beispiel 10

Ein 100 ml-Autoklav wurde unter Argon mit 24,4 g (0,1 mol) 1-Isopropyl-8-methyl-3-oxo-octandioat, mit der unter Beispiel 1 hergestellten Ru-(R)-BINAP-Katalysatorlösung und mit 40 ml sauerstofffreiem Methanol beladen. Die Hydrierung wurde bei 55°C, einem konstanten Druck von 60 bar reinem H₂ und unter intensivem Rühren 16 Stunden durchgeführt. Nach Beendigung der Reaktion wurde das Lösungsmittel am Rotationsverdampfer abdestilliert. Man erhielt 24,1 g (98%) (R)-3-Hydroxy-1-isopropyl-8-methyl-octandioat (Gehalt: 97%) mit einem Enantiomerenüberschuß von 98% (chirale HPLC).

### Beispiel 11

Ein 100 ml-Autoklav wurde unter Argon mit 25,8 g (0,1 mol) 1-Isobutyl-8-methyl-3-oxo-octandioat, mit der unter Beispiel 1 hergestellten Ru-(R)-BINAP-Katalysatorlösung und mit 40 ml sauerstofffreiem Methanol beladen. Die Hydrierung wurde bei 100°C, einem konstanten Druck von 5 bar reinem H₂ und unter intensivem Rühren 6 Stunden durchgeführt. Nach Beendigung der Reaktion wurde das Lösungsmittel am Rotationsverdampfer abdestilliert. Man erhielt 25,5 g (98%) (R)-3-Hydroxy-1-isobutyl-8-methyl-octandioat (Gehalt 96%) mit einem Enantiomerenüberschuß von 97% (chirale HPLC).

## Patentansprüche

1. Verfahren zur Herstellung von Verbindungen der allgemeinen Formel I 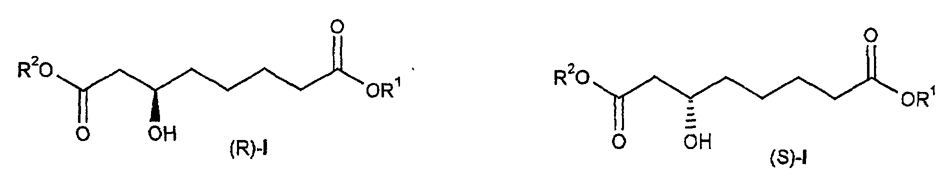 in der R¹ und R² gleich oder verschieden sind und eine C₁-C₂₀-Alkylgruppe, C₃-C₁₂-Cycloalkylgruppe, C₇-C₁₂-Aralkylgruppe oder eine ein- oder zweikernige Arylgruppe bedeuten,
**dadurch gekennzeichnet, daß** man ein Keton der Formel III 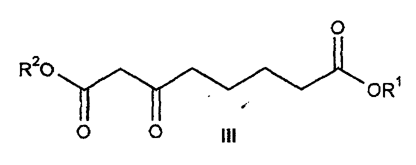 in der R¹ und R² die obige Bedeutung haben, in Gegenwart von Komplexen aus Ruthenium und optisch aktiven Phosphinen asymmetrisch hydriert.

2. Verfahren zur Herstellung von Verbindungen der allgemeinen Formel I 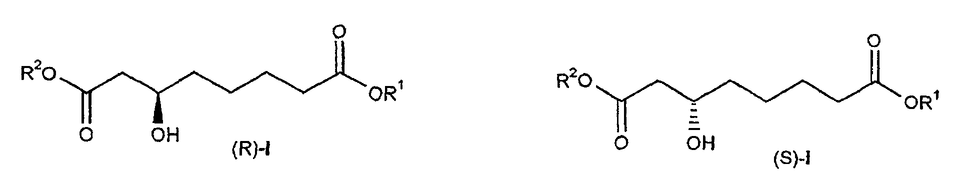 in der R¹ und R² gleich oder verschieden sind und C₁-C₂₀-Alkylgruppen, C₃-C₁₂-Cycloalkylgruppen, C₇-C₁₂-Aralkylgruppen und/oder ein- oder zweikernige Arylgruppen bezeichnen,
**dadurch gekennzeichnet, daß** man ein Keton der Formel IV 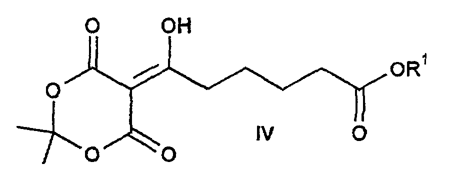 in der R¹ die obige Bedeutung hat,
in Gegenwart eines Alkohols R²OH, in der R² die obige Bedeutung hat, und von Komplexen aus Ruthenium und optisch aktiven Phosphinen asymmetrisch hydriert.

3. Verfahren gemäß Anspruch 1 oder 2, **dadurch gekennzeichnet, daß** man die asymmetrische Hydrierung in Gegenwart eines Ruthenium-Diphosphin-Komplexes der Formeln V bis XI durchführt:
[RuHal₂D]_{z}(L)ₓ V
[RuHalAD]⁺Y⁻ VI
RuDₙOOCR³OOCR⁴ VII
[RuHₓDₙ]^{m+}Yₘ⁻ VIII
[RuHal(PR⁵ ₂R⁶)D]²⁺Hal₂⁻ IX
[RuHHalD₂] X
[DRu(acac)₂] XI
worin:
acac für Acetylacetonat steht,
D für ein Diphosphin der allgemeinen Formel XII steht,
Hal für Halogen, insbesondere lod, Chlor oder Brom steht,
R³ und R⁴ gleich oder verschieden sind und für Alkyl mit bis zu 9 C-Atomen, vorzugsweise bis zu 4 C-Atomen, welches gegebenenfalls substituiert ist durch Halogen, insbesondere Fluor, Chlor oder Brom oder für Phenyl stehen, welches gegebenenfalls durch Alkyl mit 1 bis 4 C-Atomen substituiert ist oder für eine α- Aminoalkylsäure mit vorzugsweise bis zu 4 C-Atomen stehen, oder gemeinsam eine Alkylidengruppe mit bis zu 4 C-Atomen bilden,
R⁵ und R⁶ jeweils gleich oder verschieden sind und für gegebenenfalls substituiertes Phenyl stehen, vorzugsweise substituiert durch Alkyl mit 1 bis 4 C-Atomen oder Halogen,
Y für Cl, Br, J, ClO₄, BF₄ oder PF₆ stehen,
A für einen unsubstituierten oder substituierten Benzolring wie p-Cymol steht,
L für einen neutralen Liganden wie Aceton, ein tertiäres Amin oder Dimethylformamid steht,
n und m jeweils für 1 oder 2 stehen,
x für 0 oder 1 steht,
z für 1 oder 2 steht,
wobei in Formel VIII n für 1 und m für 2 steht, wenn x=0 bedeutet, und n für 2 und m für 1 steht, wenn x=1 bedeutet,
und als optisch aktive Diphosphin-Liganden D Verbindungen der allgemeinen Formel XII 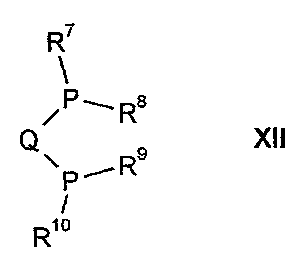 worin:
Q für eine die beiden P-Atome verbrückende Gruppe mit 2 bis 24 Kohlenstoffatomen und gegebenenfalls 1 bis 4 Heteroatomen, vorzugsweise O, S, N und Si, steht, wobei die Verbrückung von mindestens 2 der Kohlenstoffatome und gegebenenfalls 1 bis 4 der Heteroatome gebildet wird,
R⁷-R¹⁰ jeweils gleich oder verschieden sind und für Alkylgruppen mit 1 bis 18 C-Atomen, Cycloalkylgruppen mit 5 bis 7 C-Atomen oder Arylgruppen mit 6 bis 12 C-Atomen stehen,
zur Anwendung kommen.

4. Verfahren gemäß einem der Ansprüche 1 bis 3, **dadurch gekennzeichnet, daß** man die asymmetrische Hydrierung bei Temperaturen von 10°C bis 120°C unter einem Druck von 1 bis 100 bar durchführt.

5. Verfahren zur Herstellung von (R)-(+)- α-Liponsäure der Formel (R)-(+)-II 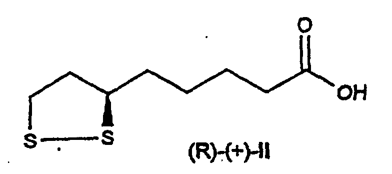 **dadurch gekennzeichnet, daß** man die Verbindungen III oder IV durch ein Verfahren gemäß Anspruch 1 oder 2 asymmetrisch zu den Verbindungen (S)-I hydriert, diese anschließend mit Natriumborhydrid in einem organischen Lösungsmittel zu den Verbindungen (S)-XIV, wobei R¹ eine
C₁-C₂₀-Alkylgruppe, C₃-C₁₂-Cycloalkylgruppe, C₇-C₁₂-Aralkylgruppe oder eine ein-oder zweikemige Arylgruppe bedeutet, reduziert 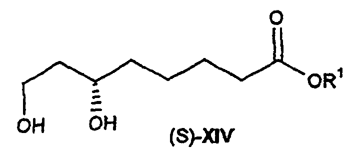 und diese
a) in organischer Lösung mit einem Sulfonsäurechlorid und einer tertiären Stickstoffbase in den Bissulfonsäureester von (S)-XIV überführt,
b) die in Schritt a) erhaltene Verbindung in einem polaren Lösungsmittel mit Schwefel und einem Alkalimetallsulfid zum (R)-(+)-α-Liponsäureester umsetzt und
c) diesen Ester in die (R)-(+)- α-Liponsäure überführt.

6. Verfahren zur Herstellung von (S)-(-)- α-Liponsäure der Formel (S)-(-)-II 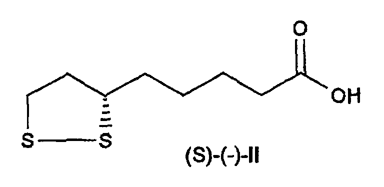 **dadurch gekennzeichnet, daß** man die Verbindungen III oder IV durch ein Verfahren gemäß Anspruch 1 oder 2 asymmetrisch zu den Verbindungen (R)-I hydriert, diese anschließend mit Natriumborhydrid in einem organischen Lösungsmittel zu den Verbindungen (R)-XIV, wobei R¹ eine
C₁-C₂₀-Alkylgruppe, C₃-C₁₂-Cycloalkylgruppe, C₇-C₁₂-Aralkylgruppe oder eine ein-oder zweikerniges Arylgruppe bedeutet, reduziert 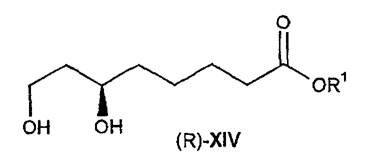 und diese
a) in organischer Lösung mit einem Sulfonsäurechlorid und einer tertiären Stickstoffbase in den Bissulfonsäureester von (R)-XIV überführt,
b) die in Schritt a) erhaltene Verbindung in einem polaren Lösungsmittel mit Schwefel und einem Alkalimetallsulfid zum (S)-(-)-α-Liponsäureester umsetzt und
c) diesen Ester in die (S)-(-)- α-Liponsäure überführt.

## Claims

1. Process for the preparation of compounds of the general formula I 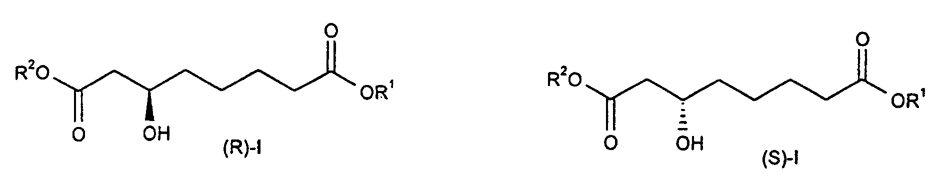 in which R¹ and R² are identical or different and are a C₁-C₂₀-alkyl group, C₃-C₁₂-cycloalkyl group, C₇-C₁₂-aralkyl group or a mono- or binuclear aryl group,
**characterized in that** a ketone of the formula III 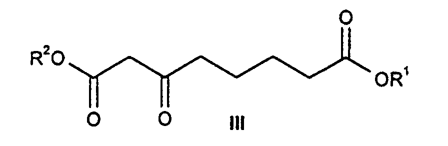 in which R¹ and R² have the above meaning,
is asymmetrically hydrogenated in the presence of complexes of ruthenium and optically active phosphines.

2. Process for the preparation of compounds of the general formula I 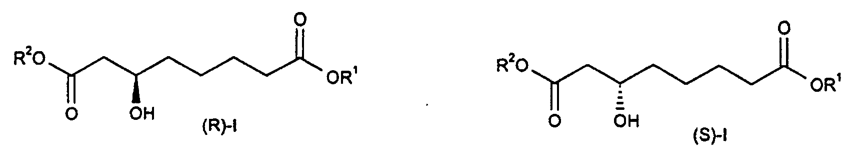 in which R¹ and R² are identical or different and are C₁-C₂₀-alkyl groups, C₃-C₁₂-cycloalkyl groups, C₇-C₁₂-aralkyl groups and/or mono- or binuclear aryl groups,
**characterized in that** a ketone of the formula IV 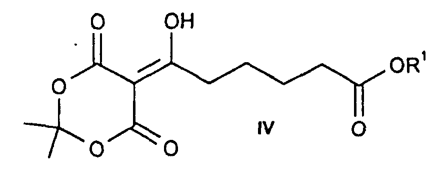 in which R¹ has the above meaning,
is asymmetrically hydrogenated in the presence of an alcohol R²OH, in which R² has the above meaning, and complexes of ruthenium and optically active phosphines.

3. Process according to Claim 1 or 2, **characterized in that** the asymmetric hydrogenation is carried out in the presence of a ruthenium-diphosphine complex of the formulae V to XI:
[RuHal₂D]_{z}(L)ₓ V
[RuHalAD]⁺Y⁻ VI
RuDₙOOCR³OOCR⁴ VII
[RuHₓDₙ]^{m+}Yₘ⁻ VIII
[RuHal (PR⁵ ₂R⁶)D]²⁺Hal₂⁻ IX
[RuHHalD₂] X
[DRu(acac)₂] XI
in which:
acac is acetylacetonate,
D is a diphosphine of the general formula XII,
Hal is halogen, in particular iodine, chlorine or bromine,
R³ and R⁴ are identical or different and are alkyl having up to 9 C atoms, preferably up to 4 C atoms, which is optionally substituted by halogen, in particular fluorine, chlorine or bromine or are phenyl which is optionally substituted by alkyl having 1 to 4 C atoms or are α-aminoalkanoic acid preferably having up to 4 C atoms, or jointly form an alkylidene group having up to 4 C atoms,
R⁵ and R⁶ in each case are identical or different and are optionally substituted phenyl, preferably substituted by alkyl having 1 to 4 C atoms or halogen,
Y is Cl, Br, I, ClO₄, BF₄ or PF₆,
A is an unsubstituted or substituted benzene ring such as p-cymene,
L is a neutral ligand such as acetone, a tertiary amine or dimethylformamide,
n and m in each case are 1 or 2,
x is 0 or 1,
z is 1 or 2,
where in formula VIII n is 1 and m is 2 if x=0, and n is 2 and m is 1 if x=1,
and as optically active diphosphine ligands D compounds of the general formula XII 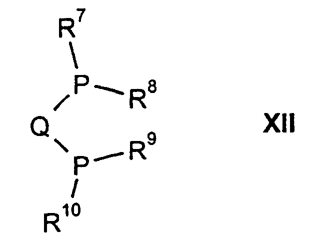 in which:
Q is a group bridging the two P atoms having 2 to 24 carbon atoms and optionally 1 to 4 heteroatoms, preferably O, S, N and Si, the bridge being formed by at least 2 of the carbon atoms and optionally 1 to 4 of the heteroatoms,
R⁷-R¹⁰ in each case are identical or different and are alkyl groups having 1 to 18 C atoms, cycloalkyl groups having 5 to 7 C atoms or aryl groups having 6 to 12 C atoms,
are used.

4. Process according to any of Claims 1 to 3, **characterized in that** the asymmetric hydrogenation is carried out at temperatures from 10°C to 120°C and under a pressure of 1 to 100 bar.

5. Process for the preparation of (R) - (+) -α-lipoic acid of the formula (R) - (+) -II 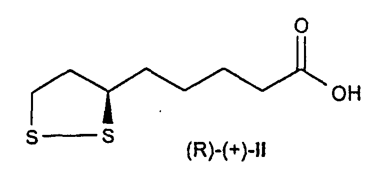 **characterized in that** the compounds III or IV are asymmetrically hydrogenated by a process according to Claim 1 or 2 to the compounds (S)-I, these are then reduced with sodium borohydride in an organic solvent to the compounds (S) -XIV, where R¹ is a C₁-C₂₀-alkyl group, C₃-C₁₂-cycloalkyl group, C₇-C₁₂-aralkyl group or a mono- or binuclear aryl group 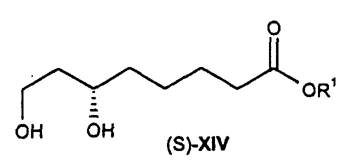 and these are converted
a) in organic solution using a sulphonyl chloride and a tertiary nitrogen base into the bis-sulphonic acid ester of (S) -XIV,
b) the compound obtained in step a) is reacted in a polar solvent with sulphur and an alkali metal sulphide to give the (R) - (+) -α-lipoic acid ester and
c) this ester is converted into the (R) - (+) -α-lipoic acid.

6. Process for the preparation of (S) - (-) -α-lipoic acid of the formula (S) - (-) -II 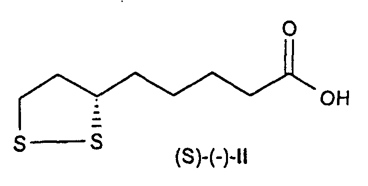 **characterized in that** the compounds III or IV are asymmetrically hydrogenated by a process according to Claim 1 or 2 to the compounds (R) -I, these are then reduced with sodium borohydride in an organic solvent to the compounds (R) -XIV, where R¹ is a C₁-C₂₀-alkyl group, C₃-C₁₂-cycloalkyl group, C₇-C₁₂-aralkyl group or a mono- or binuclear aryl group 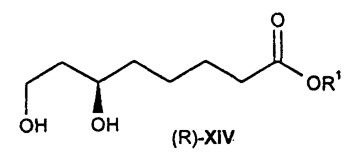 and these are converted
a) in organic solution using a sulphonyl chloride and a tertiary nitrogen base into the bis-sulphonic acid ester of (R) -XIV,
b) the compound obtained in step a) is reacted in a polar solvent with sulphur and an alkali metal sulphide to give the (S) - (-) -α-lipoic acid ester and
c) this ester is converted into the (S) - (-) -α-lipoic acid.

## Revendications

1. Procédé de préparation de composés de formule générale I 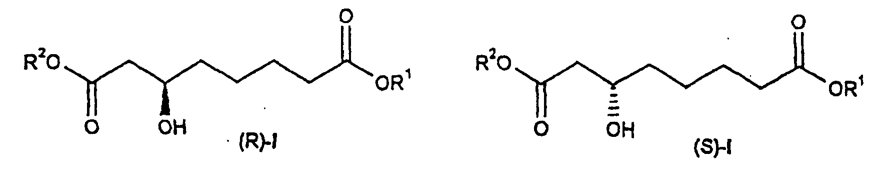 dans laquelle R¹ et R² sont identiques ou différents et représentent un groupe alkyle en C₁-C₂₀, un groupe cycloalkyle en C₃-C₁₂, un groupe aralkyle en C₇-C₁₂ ou un groupe aryle mono- ou bicyclique,
**caractérisé en ce que** l'on effectue une hydrogénation asymétrique d'une cétone de formule III 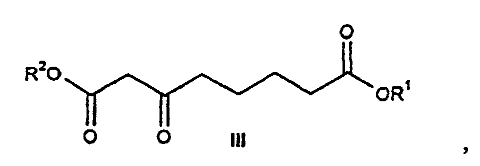 dans laquelle R¹ et R² ont la signification indiquée ci-dessus, en présence de complexes de ruthénium et de phosphines optiquement actives.

2. Procédé de préparation de composés de formule générale I 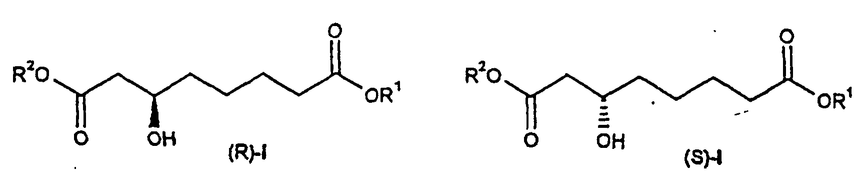 dans laquelle R¹ et R² sont identiques ou différents et représentent des groupes alkyle en C₁-C₂₀, des groupes cycloalkyle en C₃-C₁₂, des groups aralkyle en C₇-C₁₂ et/ou des groupes aryle mono- ou bicycliques,
**caractérisé en ce que** l'on effectue une hydrogénation asymétrique d'une cétone de formule IV 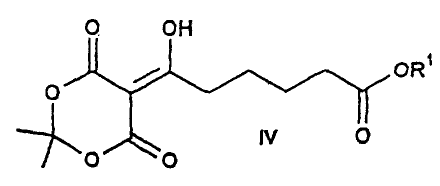 dans laquelle R¹ a la signification indiquée ci-dessus, en présence d'un alcool R²OH, où R² a la signification indiquée ci-dessus, et de complexes de ruthénium et de phosphines optiquement actives.

3. Procédé selon la revendication 1 ou 2, **caractérisé en ce que** l'on effectue l'hydrogénation asymétrique en présence d'un complexe de ruthéniumdiphosphine de formule V à XI:
[RuHal₂D]_{z}(L)ₓ V
[RuHalAD]⁺Y⁻ VI
RuDₙOOCR³OOCR⁴ VII
[RuHₓDₙ)^{m+}Yₘ⁻ VIII
[RuHal(PR⁵ ₂R⁶)D]²⁺Hal₂⁻ IX
[RuHHalD₂] X
[DRu(acac)₂] XI
où
acac représente un acétylacétonate,
D représente une diphosphine de formule générale XII,
Hal représente un halogène, en particulier l'iode, le chlore ou le brome,
R³ et R⁴ sont identiques ou différents et représentent un groupe alkyle ayant jusqu'à 9 atomes de carbone, de préférence jusqu'à 4 atomes de carbone, éventuellement substitué par un halogène, en particulier par du fluor, du chlore ou du brome, ou un groupe phényle éventuellement substitué par alkyle de 1 à 4 atomes de carbone, ou un acide α-aminoalcanoïque ayant de préférence jusqu'à 4 atomes de carbone, ou forment ensemble un groupe alkylidène ayant jusqu'à 4 atomes de carbone,
R⁵ et R⁶ sont identiques ou différents et représentent un groupe phényle éventuellement substitué, de préférence par alkyle de 1 à 4 atomes de carbone ou par halogène,
Y représente Cl, Br, I, ClO₄, BF₄ ou PF₆,
A représente un cycle benzène non substitué ou substitué comme le p-cymène,
L représente un ligand neutre comme l'acétone, une amine tertiaire ou le diméthylformamide,
n et m représentent chacun 1 ou 2,
x est 0 ou 1,
z est 1 ou 2,
et, dans la formule VIII, n est 1 et m est 2 lorsque x= 0, et n est 2 et m est 1 lorsque x = 1,
et on utilise comme ligands diphosphine optiquement actifs D des composés de formule générale XII 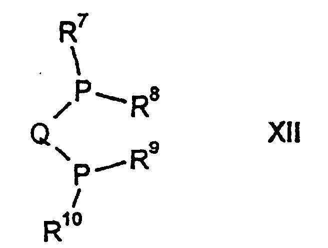 dans laquelle
Q représente un groupe de 2 à 24 atomes de carbone et éventuellement 1 à 4 hétéroatomes, de préférence O, S, N et Si, formant un pont entre les deux atomes de P, le pont étant formé d'au moins 2 des atomes de carbone et éventuellement de 1 à 4 des hétéroatomes,
R⁷-R¹⁰ sont identiques ou différents et représentent chacun un groupe alkyle de 1 à 18 atomes de carbone, un groupe cycloalkyle de 5 à 7 atomes de carbone ou un groupe aryle de 6 à 12 atomes de carbone.

4. Procédé selon l'une des revendications 1 à 3, **caractérisé en ce que** l'on effectue l'hydrogénation asymétrique à des températures de 10 à 120°C et sous une pression de 1 à 100 bars.

5. Procédé de préparation de l'acide (R)-(+)-α-lipoïque de formule (R)-(+)-II 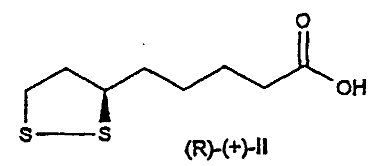 **caractérisé en ce que** l'on effectue une hydrogénation asymétrique des composés III ou IV par un procédé selon la revendication 1 ou 2 pour obtenir un composé (S)-I, puis on réduit celui-ci avec du borohydrure de sodium dans un solvant organique pour obtenir un composé (S)-XIV 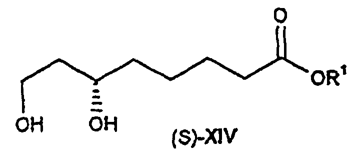 dans lequel R¹ représente un groupe alkyle en C₁-C₂₀, un groupe cycloalkyle en C₃-C₁₂, un groupe aralkyle en C₇-C₁₂ ou un groupe aryle mono- ou bicyclique, et
a) on transforme celui-ci en l'ester d'acide bissulfonique de (S)-XIV avec un chlorure d'acide sulfonique et une base azotée tertiaire dans un solvant organique,
b) on fait réagir le composé obtenu dans l'étape (a) avec du soufre et un sulfure de métal alcalin dans un solvant polaire pour obtenir l'ester d'acide (R)-(+)-α-lipoïque et
c) on transforme cet ester en l'acide (R)-(+)-α-lipoïque.

6. Procédé de préparation de l'acide (S)-(-)-α-lipoïque de formule (S)-(-)-II 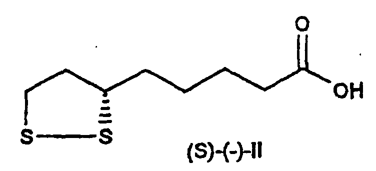 **caractérisé en ce que** l'on effectue une hydrogénation asymétrique des composés III ou IV par un procédé selon la revendication 1 ou 2 pour obtenir un composé (R)-I, puis on réduit celui-ci avec du borohydrure de sodium dans un solvant organique pour obtenir un composé (R)-XIV 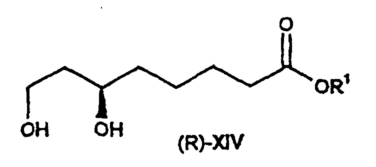 dans lequel R¹ représente un groupe alkyle en C₁-C₂₀, un groupe cycloalkyle en C₃-C₁₂, un groupe aralkyle en C₇-C₁₂ ou un groupe aryle mono- ou bicyclique, et
a) on transforme celui-ci en l'ester d'acide bissulfonique de (R)-XIV avec un chlorure d'acide sulfonique et une base azotée tertiaire dans un solvant organique,
b) on fait réagir le composé obtenu dans l'étape (a) avec du soufre et un sulfure de métal alcalin dans un solvant polaire pour obtenir l'ester d'acide (S)-(-)-α-lipoïque et
c) on transforme cet ester en l'acide (S)-(-)-α-lipoïque.
